Europäisches Patentamt

(19) European Patent Office

Office européen des brevets

(11) Veröffentlichungsnummer: **0 079 540**
**B1**

(12) **EUROPÄISCHE PATENTSCHRIFT**

(45) Veröffentlichungstag der Patentschrift:
20.03.85

(21) Anmeldenummer: **82110194.6**

(22) Anmeldetag: **05.11.82**

(51) Int. Cl.⁴: **A 61 K 7/13**

�




(54) **Haarfärbemittel.**

(30) Priorität: **13.11.81 DE 3145141**

(43) Veröffentlichungstag der Anmeldung:
**25.05.83 Patentblatt 83/21**

(45) Bekanntmachung des Hinweises auf die Patenterteilung:
**20.03.85 Patentblatt 85/12**

(84) Benannte Vertragsstaaten:
**AT BE CH DE FR GB IT LI LU NL SE**

(56) Entgegenhaltungen:
**EP - A - 0 002 828**
**DE - A - 377 288**
**DE - A - 1 907 322**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Konrad, Günter, Dr., Feuerbachweg 12, D-4010 Hilden (DE)**
Erfinder: **Maak, Norbert, Dr., Liebigstrasse 18, D-4040 Neuss (DE)**

EP 0 079 540 B1

## Beschreibung

Gegenstand der Erfindung sind Mittel zur oxidativen Färbung von Haaren, welche als Entwicklerkomponente 2,6-Dichlor-4-aminophenol enthalten und die durch Einwirkung von Dauerwellpräparaten auf Basis von Thioglycolat oder Sulfit keine Farbveränderung erleiden.

Für das Färben von Haaren werden bevorzugt sogenannte Oxidationsfarben verwendet, die durch oxidative Kupplung einer Entwicklerkomponente mit einer Kupplerkomponente entstehen, da diese meist sehr intensive Farben mit befriedigenden Echtheitseigenschaften ergeben. Als Entwicklersubstanzen werden üblicherweise Stickstoffbasen, wie z.B. p-Phenylendiaminderivate, Diaminopyridine, 4-Aminopyrazolonderivate, heterocyclische Hydrazone oder Tetraaminopyrimidine verwendet. Als sogenannte Kupplerkomponenten werden m-Phenylendiaminderivate, Phenole, Naphthole, Resorcinderivate und Pyrazolone genannt. Gute Oxidationshaarfarben müssen in erster Linie folgende Voraussetzungen erfüllen:

Sie müssen bei der oxidativen Kupplung mit den jeweiligen Entwickler- bzw. Kupplerkomponenten die gewünschten Farbnuancen in ausreichender Intensität ausbilden.

Sie müssen ferner ein gutes Aufziehvermögen auf menschlichem Haar besitzen und sollen darüber hinaus in toxikologischer und dermatologischer Hinsicht unbedenklich sein. Weiterhin ist von Bedeutung, dass auf dem zu färbenden Haar möglichst kräftige und den natürlichen Haarfarbnuancen weitgehend entsprechende Farbtöne erhalten werden. Ferner kommt der allgemeinen Stabilität der gebildeten Farbstoffe sowie deren Lichtechtheit, Waschechtheit und Thermostabilität ganz besondere Bedeutung zu, um Farbverschiebungen von der ursprünglichen Nuance oder gar Farbumschläge in andere Farbtöne zu vermeiden.

Die vorgenannten Voraussetzungen werden von Oxidationshaarfarben mit 2,6-Dichlor-4-aminophenol als Entwicklerkomponente weitgehend erfüllt. 2,6-Dichlor-4-aminophenol ist als Entwicklerkomponente von besonderem Wert, da sich mit zahlreichen bekannten Kupplerkomponenten Farbtöne von grossem Nuancenumfang und hoher Brillanz herstellen lassen. Die Erzeugung blauer Farbtöne ist vor allem zur Durchführung dunkler bis schwarzer Haarfärbungen von besonderer Wichtigkeit.

Die Verwendung von 2,6-Dichlor-4-aminophenol als Entwickler für Oxidationsfarbstoffe ist aus der DE-PS Nr. 377288 bekannt. Dort wird ein Verfahren zur Färbung von Pelzen und Haaren unter Verwendung von 4-Aminophenolen als Entwickler und 2,4-Diaminophenolethern z.B. 2,4-Diaminoanisol als Kuppler vorgeschlagen. Leider ist dieses Verfahren zum Färben von menschlichen Haaren wenig geeignet, denn es hat sich gezeigt, dass die auf diese Weise erhaltenen blauen bis schwarzvioletten Färbungen sich unter der Einwirkung von Dauerwellpräparaten, in welchen starke Reduktionsmittel, wie z.B. Thioglycolat, enthalten sind, grün verfärben. Aber auch zahlreiche sonst übliche Kupplersubstanzen, wie z.B. m-Phenylendiamin oder 2,4-Dimethyl-m-phenylendiamin ergeben mit 2,6-Dichlor-4-aminophenol blaue Färbungen, die bei der Einwirkung von Thioglycolatlösung nicht nur verblassen, sondern nach grün umschlagen.

Es stellte sich daher die Aufgabe, geeignete Kupplerkomponenten aufzufinden, die sowohl alle vorgenannten Voraussetzungen in optimaler Weise erfüllen als auch mit 2,6-Dichlor-4-aminophenol als Entwickler zu blauen Farbstoffen führen, die unter der Einwirkung von alkalischen Thioglycolatlösungen auf das gefärbte Haar allenfalls zu einer Farbaufhellung, nicht aber zu einer Grünverfärbung führen.

Es wurde nun gefunden, dass man zu Oxidationshaarfarben mit einem Gehalt an 2,6-Dichlor-4-aminophenol gelangt, welche den gestellten Anforderungen in besonders hohem Masse gerecht werden, wenn man als Kupplerkomponenten mindestens eine der Verbindungen α-Naphthol, 1,5-Dihydroxynaphthalin, m-Aminophenol und N,N-Bis-(2-hydroxyethyl)-m-phenylendiamin verwendet. Solche Oxidationshaarfarben ergeben intensive tiefblaue bis schwarzblaue Färbungen auf dem Haar, die unter dem Einfluss von Dauerwellpräparaten auf Basis von Thioglycolat zwar in Richtung blaugrau verblassen, nicht aber sich grünstichig verfärben. Die erfindungsgemäss einzusetzenden Kupplersubstanzen sind literaturbekannte Verbindungen, deren Verwendung als Kuppler für Oxidationsfarben ebenfalls an sich bekannt ist. Oxidationshaarfarben auf Basis von 2,6-Dichlor-4-aminophenol als Entwicklerkomponente und wenigstens einer der Verbindungen α-Naphthol, 1,5-Dihydroxynaphthalin, m-Aminophenol und N,N-Bis-(2-hydroxyethyl)-m-phenylendiamin lassen sich auch mit anderen sonst üblichen Entwickler- und Kupplersubstanzen zu zahlreichen Farbnuancen kombinieren, wobei die Verwendung solcher, mit 2,6-Dichlor-4-aminophenol blau kuppelnder Verbindungen, die nicht der erfinderischen Auswahl angehören, vermieden werden sollte, da dann mit unerwünschten Farbänderungen durch die Dauerwellbehandlung zu rechnen ist. Geeignete, mit 2,6-Dichlor-4-aminophenol zu braunen Farbnuancen kuppelnde Verbindungen sind z.B. Resorcin und 4-Chlorresorcin. Andere geeignete Kuppler sind z.B. dit mit 2,6-Dichlor-4-aminophenol rot kuppelnden Verbindungen 5-Amino-2-methylphenol oder 1-Phenyl-3-acetamidopyrazolon-5.

Gegenstand der Erfindung sind daher Haarfärbemittel auf Basis von Oxidationshaarfarbstoffen mit einem Gehalt an 2,6-Dichlor-4-aminophenol als Entwicklerkomponente, die als Kupplerkomponente wenigstens eine der Verbindungen α-Naphthol, 1,5-Dihydroxynaphthalin, m-Aminophenol und N,N-Bis-(2-hydroxyethyl)-m-phenylendiamin enthalten.

Ein weiterer Gegenstand der Erfindung sind Haarfärbemittel, die darüber hinaus weitere übliche Entwicklersubstanzen und übliche Kupplersubstanzen sowie gegebenenfalls übliche direktziehende Farbstoffe enthalten. Als weitere in den erfindungsgemässen Haarfärbemitteln einzusetzende Entwicklerkomponenten sind primäre aromatische Amine mit einer weiteren in p-Stellung befindlichen funktionellen Gruppe wie p-Phenylendiamin, N,N-Dimethyl-p-phenylendiamin, N,N-Diethyl-2-methyl-p-phenylendiamin, N-Ethyl-N-hydroxyethyl-p-phenylendiamin, Chlorp-phenylendiamin, N,N-Bis-hydroxyethylamino-p-phenylendiamin, Methoxy-p-phenylendiamin, 2,6-Dichlor-p-phenylendiamin, 2-Chlor-6-brom-p-phenylendiamin, 2-Chlor-6-methyl-p-phenylendiamin, 6-Methoxy-3-methyl-p-phenylendiamin, andere Verbindungen der genannten Art, die weiterhin eine oder mehrere funktionelle Gruppen wie OH-Gruppen, $NH_2$-Gruppen, NHR-Gruppen, $NR_2$-Gruppen, wobei R einen Alkyl- oder Hydroxyalkylrest mit 1 bis 4 Kohlenstoffatomen darstellt, ferner Diaminopyridinderivate, heterocyclische Hydrazonderivate wie 1-Methylpyrrolidon-(2)-hydrazon, 4-Aminopyrazolonderivate wie 4-Amino-1-phenyl-3-carbamoylpyrazolon-5, N-Butyl-N-sulfobutylp-phenylendiamin, Tetraaminopyrimidine wie 2,4,5,6-Tetraaminopyrimidin, 4,5-Diamino-2,6-bismethylaminopyrimidin, 2,5-Diamino-4-diethylamino-6-methylaminopyrimidin, 2,4,5-Triamino-6-dimethylaminopyrimidin, 2,4,5-Triamino-6-piperidinopyrimidin, 2,4,5-Triamino-6-anilinopyrimidin, 2,4,5-Triamino-6-β-hydroxyethylaminopyrimidin anzuführen.

Die als Entwicklerkomponenten oder Kupplerkomponenten in den erfindungsgemässen haarfärbemitteln einzusetzenden aromatischen Amine können entweder als solche oder in Form ihrer Salze mit anorganischen oder organischen Säuren, z.B. als Chloride, Sulfate, Phosphate, Acetate, Propionate, Lactate, Citrate eingesetzt werden.

In den erfindungsgemässen Haarfärbemitteln werden die Kupplerkomponenten im allgemeinen in etwa molaren Mengen, bezogen auf die verwendeten Entwicklersubstanzen, eingesetzt. Wenn sich auch der molare Einsatz als zweckmässig erweist, so ist es jedoch nicht nachteilig, wenn die Kupplerkomponente in einem gewissen Überschuss oder Unterschuss zum Einsatz gelangt.

Es ist ferner nicht erforderlich, dass die Entwicklerkomponente und die Kupplersubstanz einheitliche Produkte darstellen, vielmehr können sowohl die Entwicklerkomponente Gemische der erfindungsgemäss zu verwendenden Entwicklerverbindungen als auch die Kupplerkomponente Gemische der erfindungsgemäss einzusetzenden Kupplersubstanzen darstellen.

Darüber hinaus können die erfindungsgemässen Haarfärbemittel gegebenenfalls übliche direktziehende Farbstoffe im Gemisch enthalten, falls dies zur Erzielung gewisser Farbnuancen erforderlich ist.

Die oxidative Kuplung, d.h. die Entwicklung der Färbung, kann grundsätzlich, wie bei anderen Oxidationshaarfarbstoffen auch, durch Luftsauerstoff erfolgen. Zweckmässigerweise werden jedoch chemische Oxidationsmittel eingesetzt. Als solche kommen insbesondere Wasserstoffperoxid oder dessen Anlagerungsprodukte an Harnstoff, Melamin und Natriumborat sowie Gemische aus derartigen Wasserstoffperoxidanlagerungsverbindungen mit Kaliumperoxiddisulfat in Betracht.

Die erfindungsgemässen Haarfärbemittel werden für den Einsatz in entsprechende kosmetische Zubereitungen wie Cremes, Emulsionen, Gele oder auch einfache Lösungen eingearbeitet und unmittelbar vor der Anwendung auf dem Haar mit einem der genannten Oxidationsmittel versetzt. Die Konzentration derartiger färberischer Zubereitungen an Kuppler/Entwickler-Kombination beträgt 0,2 bis 5, vorzugsweise 1 bis 3 Gew.-%. Zur Herstellung von Cremes, Emulsionen oder Gelen werden die Farbstoffkomponenten mit den für derartige Präparationen üblichen Bestandteilen gemischt. Als solche zusätzlichen Bestandteile sind z.B. Netz- oder Emulgiermittel vom anionischen oder nichtionogenen Typ wie Alkylbenzolsulfonate, Fettalkoholsulfate, Alkylsulfonate, Fettsäurealkanolamide, Anlagerungsprodukte von Ethylenoxid an Fettalkohole, Verdickungsmittel wie Methylcellulose, Stärke, höhere Fettalkohole, Paraffinöl, Fettsäuren, ferner Parfümöle und Haarpflegemittel wie Pantothensäure und Cholesterin zu nennen. Die genannten Zusatzstoffe werden dabei in den für diese Zwecke üblichen Mengen eingesetzt, wie z.B. Netz- und Emulgiermittel in Konzentrationen von 0,5 bis 30 Gew.-%, jeweils bezogen auf die gesamte Zubereitung.

Die Anwendung der erfindungsgemässen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8 bis 10 erfolgen. Die Anwendungstemperaturen bewegen sich dabei im Bereich von 15 bis 40 °C. Nach einer Einwirkungsdauer von ca. 30 min wird das Haarfärbemittel vom zu färbenden Haar durch Spülen entfernt. Hernach wird das Haar mit einem milden Shampoo nachgewaschen und getrocknet.

Die nachfolgenden Beispiele sollen den Erfindungsgegenstand näher erläutern, ohne ihn jedoch hierauf zu beschränken.

*Beispiele*

In den folgenden Beispielen wurde als Entwicklersubstanz 2,6-Dichlor-4-aminophenol eingesetzt.

Als Kuppler für die erfindungsgemässen Haarfärbemittel wurden eingesetzt:

K1: α-Naphthol
K2: 1,5-Dihydroxynaphthalin
K3: m-Aminophenol
K4: N,N-Bis-(2-hydroxyethyl)-m-phenylendiamin

Zum Vergleich wurden die folgenden nicht erfindungsgemässen Blaukuppler eingesetzt:

K5: 2,4-Diaminoanisol
K6: 2,4-Diaminophenetol
K7: 2,4-Diaminobutoxybenzol
K8: m-Phenylendiamin
K9: 2,4-Dimethyl-m-Phenylendiamin

Die Haarfärbemittel wurden in Form einer Cremeemulsion eingesetzt. Dabei wurden in eine Emulsion aus 10 Gew.-Teilen Fettalkoholen der Kettenlänge $C_{12}$-$C_{18}$, 10 Gew.-Teilen Fettalkoholsulfat (Natriumsalz) der Kettenlänge $C_{12}$-$C_{18}$, 75 Gew.-Teilen Wasser jeweils 0,01 mol der in der nachstehenden Tabelle aufgeführten Entwickler- und Kupplersubstanzen eingearbeitet. Danach wurde der pH-Wert der Emulsion mittels Ammoniak auf 9,5 eingestellt und die Emulsion mit Wasser auf 100 Gew.-Teile aufgefüllt. Die oxidative Kupplung wurde mit 1%iger Wasserstoffperoxidlösung als Oxidationsmittel durchgeführt, wobei zu 100 Gew.-Teilen der Emulsion 10 Gew.-Teile Wasserstoffperoxidlösung gegeben wurden. Die jeweilige Färbecreme mit zusätzlichem Oxidationsmittel wurde auf zu 90% ergrautes, nicht besonders vorbehandeltes Menschenhaar aufgetragen und dort 30 min gelassen. Nach Beendigung des Färbeprozesses wurde das Haar mit einem üblichen Haarwaschmittel ausgewaschen und anschliessend getrocknet. Die dabei erhaltenen Färbungen sind nachstehender Tabelle zu entnehmen (vor Dauerwellbehandlung).

Die getrockneten, gefärbten Haarproben wurden anschliessend mit einem marktüblichen Dauerwellpräparat, welches 8 Gew.-% Ammoniumthioglycolat, 5 Gew.-% Ammoniumcarbonat, 6 Gew.-% nichtionogenen Emulgator sowie freies Ammoniak in wässeriger Lösung enthielt und einen pH-Wert von 8,8 aufwies, bei 77 °C 30 min lang behandelt. Danach wurden die Haarproben gewaschen und mit einer marktüblichen wässerigen Fixierlösung, welche 3,5 Gew.-% Kaliumbromat neben ca. 8 Gew.-% Netz- und Emulgiermitteln enthielt, bei 20 °C 10 min lang behandelt.

Die durch diese Behandlung erzielte Verblassung bzw. Veränderung der Färbungen ist ebenfalls der Tabelle zu entnehmen (nach Dauerwellbehandlung).

### Tabelle

| Beispiel | Kuppler | vor Dauerwellbehandlung | nach Dauerwellbehandlung |
|---|---|---|---|
| 1 | K1 | tintenblau | graublau |
| 2 | K2 | blau | graublau |
| 3 | K3 | dunkelblau | graublau |
| 4 | K4 | blau | graublau |
| 5 | K5 | schwarzblau | mattgrün |
| 6 | K6 | schwarzblau | mattgrün |
| 7 | K7 | schwarzblau | mattgrün |
| 8 | K8 | schwarzblau | mattgrün |
| 9 | K9 | schwarzblau | mattgrün |

## Patentansprüche

1. Haarfärbemittel auf Basis von Oxidationshaarfarbstoffen mit einem Gehalt an 2,6-Dichlor-4-aminophenol als Entwicklerkomponente, dadurch gekennzeichnet, dass als Kupplerkomponente mindestens eine der Verbindungen α-Naphthol, 1,5-Dihydroxynaphthalin, m-Aminophenol und N,N-Bis-(2-hydroxyethyl)-m-phenylendiamin enthalten ist.

2. Haarfärbemittel nach Anspruch 1, gekennzeichnet durch einen Gehalt weiterer, üblicher Entwicklersubstanzen und üblicher Kupplersubstanzen sowie gegebenenfalls üblicher direktziehender Farbstoffe.

3. Haarfärbemittel nach den Ansprüchen 1 und 2, gekennzeichnet durch einen Gehalt an Entwickler/Kuppler-Kombination von 0,2 bis 5, vorzugsweise von 1 bis 3 Gew.-%, bezogen auf das gesamte Mittel.

## Claims

1. Hair-dyeing compositions based on oxidation hair-dyes containing 2,6-dichloro-4-aminophenol as developer component, characterized in that at least one of the compounds α-naphthol, 1,5-dihydroxynaphthaline, m-aminophenol and N,N-bis-(2-hydroxyethyl)-m-phenylene diamine is present as the coupler component.

2. Hair-dyeing compositions as claimed in Claim 1, characterized by a content of other standard developer substances and standard coupler substances and, optionally, standard substantive dyes.

3. Hair-dyeing compositions as claimed in Claims 1 and 2, characterized by a content of developer/coupler combination of from 0.2 to 5% by weight and preferably from 1 to 3% by weight, based on the composition as a whole.

## Revendications

1. Compositions pour la teinture des cheveux, à base de colorants d'oxydation pour cheveux, contenant du 2,6-dichloro-4-aminophénol comme composant de développement, compositions caractérisées en ce que, comme composé de copulation, elles contiennent au moins un des composés α-naphtol, 1,5-dihydroxynaphtaline, m-aminophénol et N,N-bis-(2-hydroxyéthyl)-m-phénylènediamine.

2. Compositions selon la revendication 1, caractérisées en ce qu'elles contiennent d'autres substances usuelles de développement et d'autres substances usuelles de copulation, ainsi qu'éventuellement des colorants directs.

3. Compositions selon les revendications 1 et 2, caractérisées en ce qu'elles contiennent 0,2 à 5% en poids, de préférence 1 à 3% en poids, de combinaison de développement de copulation.